# EUROPEAN PATENT SPECIFICATION

(11) **EP 4 120 976 B1**
(45) Date of publication and mention of the grant of the patent: **19.11.2025**
(21) Application number: 21712237.3
(22) Date of filing: 11.03.2021
(51) Int. Cl.: A61F 9/007, A61F 9/008

(54) **LASER VITRECTOMY AND ILLUMINATION PROBE**
LASERVITREKTOMIE UND BELEUCHTUNGSSONDE
VITRECTOMIE LASER ET SONDE D'ÉCLAIRAGE

(30) Priority: 19.03.2020 US 202062991639 P
(43) Date of publication of application: 25.01.2023
(73) Proprietor: Alcon Inc., 1701 Fribourg (CH)
(72) Inventor: HALLEN, Paul R., Fort Worth, Texas 76134 (US)
(74) Representative: Murgitroyd & Company
(86) International application number: PCT/IB2021/052048
(87) International publication number: WO 2021/186305

(56) References cited:
- US-A1- 2008 108 983
- US-A1- 2012 041 461
- US-A1- 2019 201 238

## Description

### BACKGROUND

### Field

Embodiments of the present disclosure generally relate to small-gauge instrumentation for surgical procedures, and more particularly, small-gauge instrumentation for laser vitreous surgery.

### Description of the Related Art

Anatomically, the human eye is divided into two distinct regions- the anterior segment and the posterior segment. The anterior segment includes the lens and extends from the outermost layer of the cornea to the posterior of the lens capsule. The posterior segment of the eye includes the anterior hyaloid membrane and all of the ocular structures behind it, such as the vitreous humor, retina, choroid, and the optic nerve.

Vitreoretinal procedures are commonly performed within the posterior segment of the human eye to treat serious conditions such as age-related macular degeneration (AMD), macular holes, premacular fibrosis, retinal detachment, epiretinal membrane, cytomegalovirus (CMV) retinitis, diabetic retinopathy, vitreous hemorrhages, and other ophthalmic conditions. Such procedures frequently require the severance and removal of portions of the vitreous humor from the posterior segment of the eye, which is a colorless and gel-like substance that makes up approximately two-thirds of the eye's volume. In a vitrectomy procedure, a surgeon inserts microsurgical instruments through one or more incisions made in the eye to cut and remove the vitreous body from within. A separate incision may be provided for each microsurgical instrument when using multiple instruments simultaneously.

The microsurgical instruments typically utilized during a vitrectomy include a vitrectomy probe for severing and removing the vitreous body and an illumination probe to provide illumination within the intraocular space. Proper illumination of the intraocular space is advantageous in order for a surgeon to adequately view the vitreous body, to the extent possible, for purposes of removal with the vitrectomy probe. In certain cases, to reach the vitreous body located at peripheral regions of the eye, surgeons may also utilize a scleral depressor to displace the retina inward, in addition to other microsurgical instruments. Thus, during any given vitrectomy procedure, three or more microsurgical instruments may be simultaneously used, whereas the surgeon only has two hands to perform the procedure. Reference is made to US 2019/201238 A1, US 2012/041461 A1 and US 2008/108983 A1 which have been cited as relating to the state of the art. US 2012/041461 for example provides in an exemplary arrangement a probe having an endo-laser optical fiber located in a lumen of a cannula and illumination fibers arranged circumferentially around and encircling the cannula.

### SUMMARY

The scope is in accordance with the appended claims.

The present disclosure generally relates to microsurgical instruments for ophthalmic surgical procedures, and more particularly, microsurgical instruments having combined illumination and laser vitrectomy functions.

In one embodiment, a surgical instrument is provided. The surgical instrument includes a base unit and a probe disposed through an opening in a distal end of the base unit. The probe further includes a port formed in a sidewall of the probe and proximate to a distal tip of the probe, a lumen formed through the probe, and one or more optical fibers disposed in the lumen. The one or more optical fibers comprising a first optical fiber configured to project a laser light produced by a laser light source for irradiating an area proximate to the port to cut collagen fibers of vitreous material aspirated through the port, the one or more optical fibers further comprising a second optical fiber configured to project an illumination light produced by an illumination light source for illumination of an intraocular space of a patient; and
wherein the first optical fiber and the second optical fiber are each coupled to the sidewall of he lumen and are aligned with the port.

### BRIEF DESCRIPTION OF THE DRAWINGS

So that the manner in which the above recited features of the present disclosure can be understood in detail, a more particular description of the disclosure, briefly summarized above, may be had by reference to embodiments, some of which are illustrated in the appended drawings. It is to be noted, however, that the appended drawings illustrate only exemplary embodiments and are therefore not to be considered limiting of its scope, and may admit to other equally effective embodiments.
Figure 1 illustrates a perspective view of an exemplary surgical instrument according to some embodiments of the present disclosure.
Figure 2A illustrates a plan view of a portion of the surgical instrument of Figure 1 according to some embodiments of the present disclosure.
Figure 2B illustrates a stylized longitudinal cross-sectional view of a portion of the surgical instrument of Figure 1 according to some embodiments of the present disclosure.
Figure 2C illustrates a stylized longitudinal cross-sectional view of a portion of the surgical instrument of Figure 1 according to some embodiments of the present disclosure.
Figure 2D illustrates a longitudinal schematic view of a portion of the surgical instrument of Figure 1 according to some embodiments of the present disclosure.
Figure 3A illustrates a front sectional view of an exemplary surgical instrument according to some embodiments of the present disclosure.
Figure 3B illustrates a front sectional view of an exemplary surgical instrument according to some embodiments of the present disclosure.
Figure 3C illustrates a front sectional view of an exemplary surgical instrument according to some embodiments of the present disclosure.
Figure 4A illustrates a front sectional view of an exemplary surgical instrument according to some embodiments of the present disclosure.
Figure 4B illustrates a front sectional view of an exemplary surgical instrument according to some embodiments of the present disclosure.
Figure 4C illustrates a front sectional view of an exemplary surgical instrument according to some embodiments of the present disclosure.
Figure 4D illustrates a front sectional view of an exemplary surgical instrument according to some embodiments of the present disclosure.
Figure 5A illustrates a front sectional view of an exemplary surgical instrument according to some embodiments of the present disclosure.
Figure 5B illustrates a front sectional view of an exemplary surgical instrument according to some embodiments of the present disclosure.
Figure 6A illustrates a plan view of a portion of an exemplary surgical instrument according to some embodiments of the present disclosure.
Figure 6B illustrates a plan view of a portion of an exemplary surgical instrument according to some embodiments of the present disclosure.
Figure 6C illustrates a plan view of a portion of an exemplary surgical instrument according to some embodiments of the present disclosure.
Figure 6D illustrates a plan view of a portion of an exemplary surgical instrument according to some embodiments of the present disclosure.

To facilitate understanding, identical reference numerals have been used, where possible, to designate identical elements that are common to the figures. It is contemplated that elements and features of one embodiment may be beneficially incorporated in other embodiments without further recitation.

### DETAILED DESCRIPTION

The present disclosure generally relates to microsurgical instruments for ophthalmic surgical procedures, and more particularly, microsurgical instruments having combined illumination and laser vitrectomy functions. In some embodiments, a surgical instrument includes a base and a probe having a main lumen and a port at a distal tip thereof. In some embodiments, the probe may further include a single optical fiber within the main lumen, the single optical fiber configured to project both laser light as well as illumination light. According to some embodiments, when (e.g., as soon as) vitreous material is drawn into the probe, e.g., through the port, the vitreous material passes through a volume irradiated by the laser light emitted by the optical fiber, thus severing the vitreous material. Simultaneously, the illumination light provides enhanced visualization of the intraocular space during severance and removal of the vitreous material. In some other embodiments, separate optical fibers may be used for projecting laser and illumination lights. For example, in such embodiments, a first optical fiber may be used for projecting laser light while one or more additional optical fibers may be used to project illumination light.

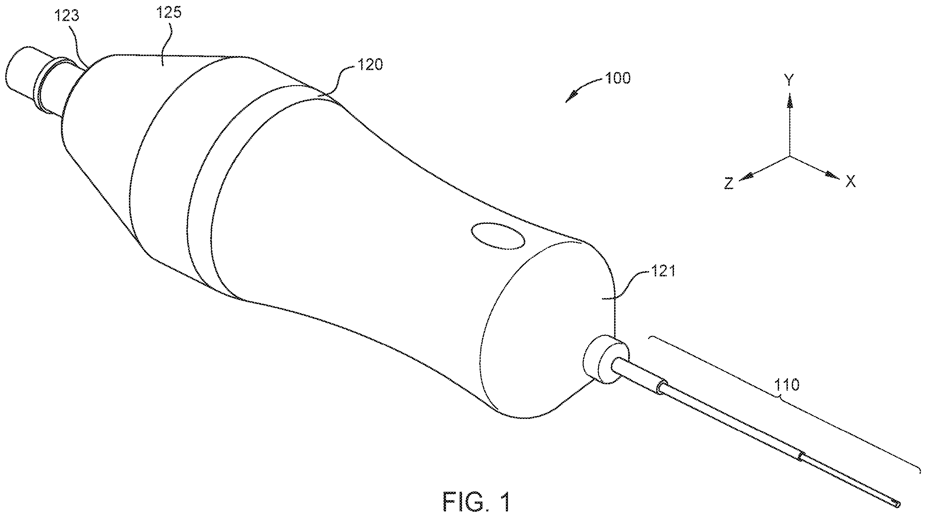

Figure 1 illustrates a perspective view of an exemplary surgical instrument 100 according to certain embodiments described herein. As depicted in Figure 1, the surgical instrument 100 comprises a probe 110 and a base unit 120. The probe 110 is partially and longitudinally disposed through a distal end 121 of the base unit 120 and may be directly or indirectly attached thereto within an interior chamber of the base unit 120. Note that, as described herein, a distal end or portion of a component refers to the end or the portion that is closer to a patient's body during use thereof. On the other hand, a proximal end or portion of the component refers to the end or the portion that is distanced further away from the patient's body.

In some embodiments, the base unit 120 is a hand piece having an outer surface configured to be held by a user, such as a surgeon. For example, the base unit 120 may be ergonomically contoured to substantially fit the hand of the user. In some embodiments, the outer surface may be textured or have one or more gripping features formed thereon, such as one or more grooves and/or ridges. The base unit 120 may be made from any materials commonly used for such instruments and suitable for ophthalmic surgery. For example, the base unit 120 may be formed of a lightweight aluminum, a polymer, or other suitable material. In some embodiments, the base unit 120 may be sterilized and used in more than one surgical procedure, or it may be a single-use device.

The base unit 120 further provides one or more ports 123 (e.g., one port 123 is depicted in Figure 1) at a proximal end 125 thereof for one or more supply lines to be routed into an interior chamber of the base unit 120. For example, the port 123 may provide a connection between the base unit 120 and a vacuum line of a vacuum source for aspiration. The port 123 may also provide a connection to an optical fiber cable that couples to one or more light sources for providing laser light as well as illumination light.

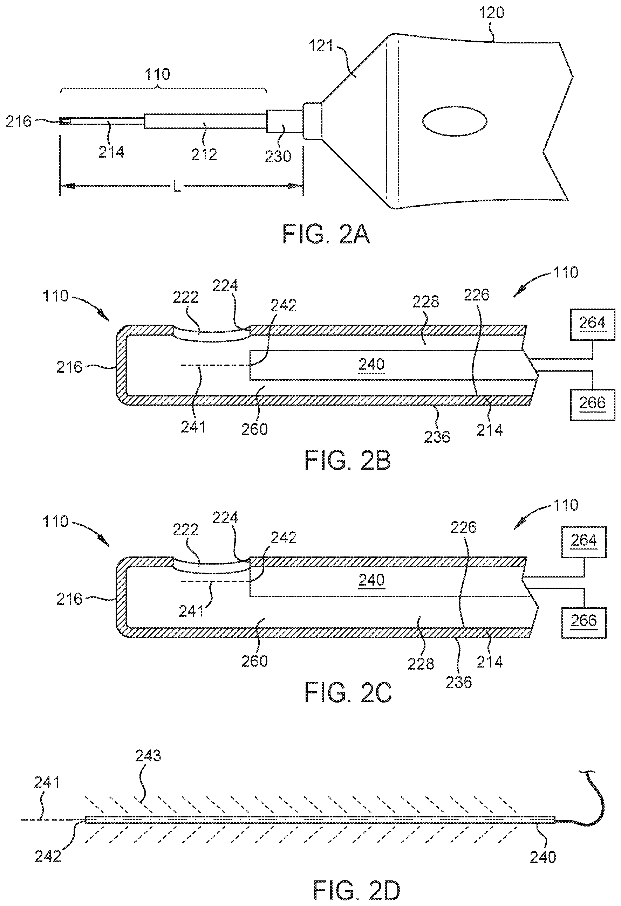

Figure 2A illustrates a plan view of the probe 110 and the distal end 121 of the base unit 120. As shown, the probe 110 may be an elongated laser cutting member that may be inserted into an eye, e.g., through an insertion cannula, for performing vitrectomy, which may be aspirating or non-aspirating. The probe 110 may thus be formed of materials suitable for minimally invasive vitreoretinal surgeries. In some embodiments, the probe 110 includes one or more sections that are formed of materials configured to transmit laser light, visible light, ultraviolet light, infrared light, or any other type of light. For example, the probe 110 may include one or more sections formed of a translucent or transparent material, such as a plastic and/or polymeric material. The probe 110 may further include one or more sections formed of more conventional surgical-grade materials, such as stainless steel and/or aluminum.

In certain embodiments, the probe 110 has a length L between about 15 mm (millimeters) and about 30 mm, but may have a larger or smaller length in some embodiments. The probe 110 may comprise a hollow tube having an outer diameter less than about 20 gauge. In some embodiments, the probe 110 is segmented into two or more portions (e.g., regions or segments) having outer diameters of differing sizes. For example, as shown in Figure 2A, the probe 110 may include a proximal portion 212 having a larger outer diameter than a distal portion 214 that terminates at a distal tip 216. In some embodiments, the proximal portion 212 has an outer diameter of about 23 gauge and the distal portion 214 has an outer diameter of about 25 gauge. In some embodiments, the proximal portion 212 has an outer diameter of about 25 gauge and the distal portion 214 has an outer diameter of about 27 gauge. In some other embodiments, the proximal portion 212 has an outer diameter of about 27 gauge and the distal portion 214 has an outer diameter of about 29 gauge. In some embodiments, the proximal portion 212 functions as an infusion portion and is configured to direct infusion fluid into the operating space adjacent the probe during use thereof. The proximal portion 212 may thus include one or more coaxial infusion ports concentrically disposed around the distal portion 214 and fluidly connected to a fluid source through the base unit 120. Delivery of infusion fluid to the interior eye during vitreoretinal surgery enables the maintenance of intraocular pressure, thereby preventing the eye from collapsing during the surgical procedure.

In some embodiments, the surgical instrument 100 further includes a stiffener 230 fixedly or slidably coupled to and substantially surrounding at least a portion of the probe 110. For example, the stiffener 230 is slidably coupled to an exterior surface 236 (shown in Figures 2B-2C) of the probe 110 and may extend from and retract into the base unit 120. The stiffener 230 may be adjustable relative to the probe 110, enabling a user to position the stiffener 230 at different points along a length L of the probe 110 exterior to the base unit 120. Accordingly, a user may selectively adjust the level of stiffness of the probe 110 by repositioning the stiffener 230 relative to the distal tip 216, thereby manipulating the amount of support provided to the probe 110 and stabilizing the surgical instrument 100 during use thereof.

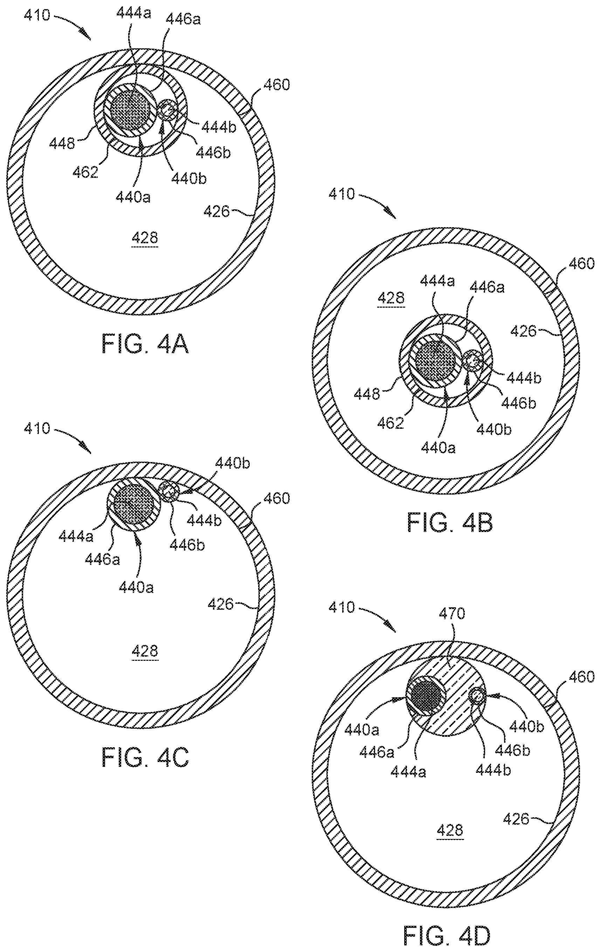

As described above, in some embodiments, the surgical instrument 100 provides a single optical fiber that is configured to project both laser light as well as illumination light. Various examples of using a single optical fiber for projecting both laser light and illumination light are depicted in Figures 2B-3C. In some other embodiments, one or more optical fibers may be used for projecting laser light while one or more additional optical fibers may be used for projecting illumination light. Various examples of using multiple fibers for projecting laser light and illumination light are depicted in Figures 4A-5B.

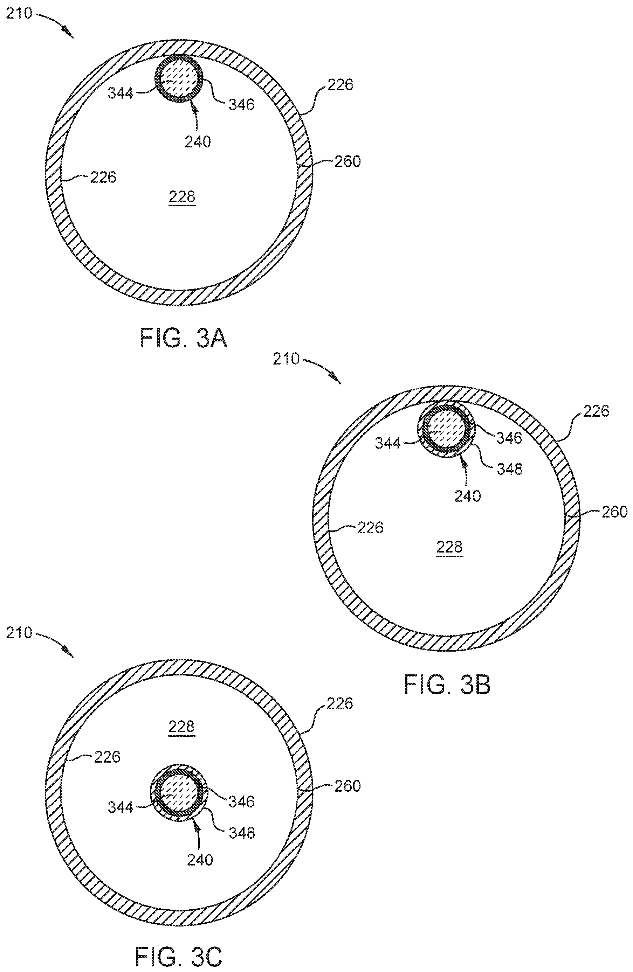

Figures 2B and 2C illustrate stylized longitudinal cross-sectional views of the distal portion 214 of the probe 110 having the optical fiber 240 housed therein. As depicted, the probe 110 includes a main lumen 260 and a port 222 near the distal tip 216. In one example, the main lumen 260 has a substantially circular cross section, shown in Figures 3A-5B. The port 222, which is located at the distal tip 216 of the distal portion 214, is sized and shaped to allow vitreous collagen fibers to enter the main lumen 206 during vitrectomy. In some examples, the vitreous collagen fibers may be aspirated into the main lumen 206 through the port 222. As further described below, the optical fiber 240 is configured to project a laser light 241 to sever the vitreous fibers that enter the port 222.

The optical fiber 240 may be designed to operate as an optical waveguide and propagate the laser light 241 through a terminal end 242 thereof. The characteristics of the laser light 241 propagated through the optical fiber 240 are such that the laser light 241 causes disruption of the vitreous collagen fibers within the path of the laser light 241. Disruption refers to the breaking down of the tissue by rapid ionization of molecules thereof. In some examples, the laser light 241 may be produced by a laser light source 264 optically coupled to the optical fiber 240 using an optical fiber cable, as described above. In some embodiments, the laser light 241 propagated by the optical fiber 240 is an ultraviolet ("UV") (<350 nm) laser light. In other embodiments, the laser light 241 is an argon blue-green laser light (488 nm), a Nd-YAG laser light (532 nm) such as a frequency-doubled Nd-YAG laser light, a krypton red laser light (647 nm), a diode laser light (805-810nm), or any other suitable type of laser light for ophthalmic surgery.

In some embodiments, the laser light source 264 may produce a laser light 241 having a pulse rate within a range of about 10 kilohertz (kHz) and about 500 kHz. This range can effectively provide disruption of the vitreous body. Other pulse rate ranges can also provide disruption and are thus contemplated as well. In some examples, the laser light source 264 produces a picosecond or femtosecond laser light 241. In some embodiments, the laser light source 264 may produce a continuous coherent laser light 241. For example, the laser light source 264 may produce a continuous coherent laser light 241 at low power.

In certain embodiments, the optical fiber 240 is disposed within the main lumen 260 and terminates at the terminal end 242 near the port 222 such that the laser light 241 projecting from the optical fiber 240 will be projected across the port 222 with sufficient power to sever vitreous collagen fibers. In the embodiment depicted in Figure 2B, the optical fiber 240 is rigidly suspended within the main lumen 260 such that the optical fiber 240 is separated from an interior sidewall 226 of the probe 110 and the optical fiber 240 is circumferentially surrounded by a space 228. The space 228 formed between the optical fiber 240 and the interior sidewall 226 of the probe 110 provides a coaxial path for aspiration of severed vitreous collagen fibers through the probe 110. In some embodiments, the optical fiber 240 may be centrally disposed within the main lumen 260 such that a radial distance between the interior sidewall 226 and the optical fiber 240 is uniform along a circumference of the optical fiber 240.

In the embodiment depicted in Figure 2C, the optical fiber 240 may be disposed along (e.g., coupled to) the interior sidewall 226. For example, the optical fiber 240 may be coupled to the interior sidewall 226 along a longitudinal length thereof. In some embodiments, the optical fiber 240 is coupled to the interior sidewall 226 along a portion of the interior sidewall 226 radially aligned with the port 222 such that the terminal end 242 of the optical fiber 240 terminates at a point radially inward of the port 222 relative to a longitudinal central axis of the probe 110. The optical fiber 240 may be coupled to the interior sidewall 226 with any suitable adhesive or bonding mechanism, such as an epoxy or acrylic adhesive.

The terminal end 242 of the optical fiber 240 may terminate at any point along the length L of the probe 110 to enable optimal severance of vitreous fibers as well as aspiration thereof. In some embodiments, the terminal end 242 of the optical fiber 240 terminates within the main lumen 260 at a point distal to a proximal end 224 of the port 222. In other embodiments, the terminal end 242 of the optical fiber 240 terminates at a point within the main lumen 260 substantially aligned with the proximal end 224. In still other embodiments, the terminal end 242 of the optical fiber 240 terminates within the main lumen 260 at a point proximal to the proximal end 224.

In some embodiments, the laser light 241 projected by the optical fiber 240 has a diameter or width substantially smaller than a diameter or width of the port 222. For example, the port 222 may have a diameter or width between about 200 µm (micrometres) and about 500 µm, such as between about 250 µm and about 450 µm, such as about 300 µm. The laser light 241 may have a diameter or width between about 5 µm and about 50 µm, such as between about 10 µm and about 40 µm, such as between about 15 µm and about 30 µm. In some examples, the laser light 241 projected by the optical fiber 240 is scanned across the port 222.

In the embodiments of Figures 2B-2D, the optical fiber 240 is further configured to propagate illumination light 243 (shown in Figure 2D) in addition to and separate from the laser light 241. For example, an illumination light source 266 may be used to provide illumination light 243 to the optical fiber 240. The optical fiber 240 propagates the illumination light 243 in one of a variety of ways to illuminate the intraocular space. An example of an illumination light source 266 includes a UV (ultraviolet) light source, a violet light source, a blue light source, a white light source, an infrared ("IR") light source, or any other suitable type of illumination light source. For example, an LED-based (light-emitting diode based) illumination light source 266 may be utilized. In further examples, a xenon- or halogen-based illumination light source 266 may be utilized.

In embodiments where a single optical fiber 240 is used for projecting both laser light as well as illumination light, the laser light source 246 may be configured to focus the laser light 241 on a core of the optical fiber 240 and thus, the laser light 241 is transmitted through the core. In some embodiments, the illumination light source 266 is configured to focus the illumination light 243 onto both the core and a cladding of the optical fiber 240, in which case both the cladding and the core transmit the illumination light 243. In yet some other embodiments, the illumination light source 266 is configured to focus the illumination light 243 onto just the core or the cladding, in which case only one of the core or the cladding transmit the illumination light 243. Thus, the optical fiber 240, including a core and a cladding, is capable of transmitting the laser light 241 (through the core) and the illumination light 243 (through the cladding and the core) in the same fiber. In some embodiments, the illumination light 243 is propagated through one or more additional cores in the optical fiber 240. Thus, the optical fiber 240 may include one or more cores through which the laser light 241 and the illumination light 243 are separately propagated.

In some embodiments, the illumination light 243 is coaxially projected with the laser light 241 from the terminal end 242 of the optical fiber 240. In some embodiments, the illumination light 243 is diffusely projected from the terminal end 242. In some embodiments, the illumination light 243 undergoes total internal reflection within the optical fiber 240, and thus, is only projected from the terminal end 242. For example, the optical fiber 240 is an end-emitting optical fiber. In some embodiments, the illumination light 243 is not completely reflected within the optical fiber 240 and may be emitted through a sidewall of a cladding alternatively or in addition to the terminal end 242. For example, the optical fiber 240 is an edge-emitting or side-emitting optical fiber, where illumination light 243 is emitted radially outward therefrom. The illumination light 243 is propagated simultaneously with the laser light 241 or sequentially pulsed with the laser light 241. In certain embodiments, propagation of illumination light 243 through the optical fiber 240 and into the intraocular space may be modulated by utilizing different types of illumination light sources 266, utilizing different materials for the optical fiber 240, modifying the physical arrangement of the optical fiber 240 within the probe 110, and/or by utilizing different materials for the probe 110.

Figure 2D illustrates an example of optical fiber 240 propagating both a laser light 241 and an illumination light 243 therefrom. In the example of Figure 2D, the laser light 241 is emitted through the terminal end 242 of the optical fiber 240 while the illumination light 243 is emitted radially outward from the optical fiber 240. In other words, in the example of FIG. 2D, the laser light 241 is focused onto a core of the optical fiber 240 while the illumination light 243 is focused onto a cladding of the optical fiber 240. However, as described above, in some other embodiments, the illumination light 243 may instead be focused on both the cladding and the core of the optical fiber 240. In such embodiments, the illumination light 243 is not only emitted radially but also through the terminal end 242 of the optical fiber 240. In still other embodiments, the illumination light 243 is focused onto only the core of the optical fiber 240.

In some embodiments, the optical fiber 240 is communicatively coupled to a digital visualization system, such as the NGENUITY^{®} 3D Visualization System produced by Alcon. Other digital visualization systems, including those produced by other manufacturers, are also contemplated for use with the embodiments described herein. Utilization of a digital visualization system may enable modification of the color and intensity of illumination light 243 emitted from the optical fiber 240 by adjustment of hue, saturation, gamma, tint, and/or other light parameters.

While "light" is discussed herein, the scope of the disclosure is not intended to be limited to visible light. Rather, other types of radiation, such as UV and IR radiation, may be transmitted from the optical fiber 240, and the term "light" is intended to encompass all types of radiation for use with the optical fiber 240. In some examples, non-visible light may be transmitted by the optical fiber 240 and captured by non-visible light sensors for analysis with the digital visualization systems described above. Thus, a non-visible light source may be coupled to the optical fiber 240 in addition to an illumination light source 266 and/or a laser light source 264, and the non-visible light may be propagated simultaneously with or sequentially pulsed with the laser light 241 and the illumination light 243.

In some embodiments, the optical fiber 240 has a diameter between about 20 µm and about 120 µm, such as a diameter between about 40 µm and about 100 µm. For example, the optical fiber 240 has a diameter between about 50 µm and about 80 µm. However, smaller or larger diameters are also contemplated. In some embodiments, a light sleeve assembly containing a plurality of optical fibers 240 is utilized. For example, a light sleeve containing a plurality of optical fibers 240 having uniform or different diameters may be utilized. In further embodiments, the optical fiber 240 is a multi-mode end-emitting fiber, a single-mode end-emitting fiber, or the like.

Figures 3A-3C illustrate exemplary front sectional views of the probe 210 of Figures 2A-2D, having the optical fiber 240 housed therein for projecting both laser and illumination lights. As depicted, the probe 210 has a circular cross section defined by the interior sidewall 226 and the exterior surface 236. Generally, the optical fiber 240 includes a core 344 and a cladding 346 circumferentially surrounding the core 344 in accordance with embodiments of the present disclosure. The core 344 may comprise any transparent material, such as fused silica or glass. In some embodiments, the core 344 is doped. For example, the core 344 may be germanium-doped silica. Doping the core 344 with germanium or a similar dopant may increase the refractive index of the core 344 compared to that of the cladding 346 material, hence enabling laser and light guiding properties within the core 344.

The cladding 346 may also comprise a transparent material, such as fused silica or glass. In some embodiments, the cladding 346 is doped in addition to or instead of doping the core 344. For example, the cladding 346, which may comprise fused silica, is doped with a dopant that reduces the refractive index of the cladding 346 relative to that of the core 344. Example dopants include fluorine (F), chlorine (CI), boron (B), or the like. The cladding 346, when doped, has a lower refractive index than the core 344, thus enabling light guiding properties within the core 344. Although one cladding 346 is depicted in each of Figures 3A-3C, the optical fiber 340 may further include one or more additional claddings.

In one example, the core 344 has a diameter in the range of 5 µm and about 100 µm, such as a diameter between about 20 µm and about 80 µm, such as a diameter of about 75 µm. However, smaller or larger diameters are also contemplated. In one example, the cladding 346 has a thickness between about 5 µm and about 50 µm, such as a thickness between about 15 µm and about 40 µm, such as a thickness of about 25 µm. However, smaller or larger thicknesses are also contemplated.

In some embodiments as depicted in Figures 3B and 3C, the optical fiber 240 is disposed within a sleeve 348. The sleeve 348 may couple directly or indirectly to an exterior of the cladding 346 and circumferentially surround the cladding 346 and the core 344 of the optical fiber 240 therewithin. The sleeve 348 may act as a tubular structure for providing structural support and alignment of the optical fiber 240 within the main lumen 260 of the probe 210. Similar to the core 344 and the cladding 346, the sleeve 348 may comprise a transparent material such as fused silica and glass. In further embodiments, the sleeve 348 is doped with a dopant to manipulate the refractive index of the sleeve 348 as desired. The sleeve 348 may have a thickness between about 5 µm and about 50 µm, such as a thickness between about 15 µm and about 40 µm, such as a thickness of about 25 µm. However, smaller or larger thicknesses are also contemplated. In embodiments where the sleeve 348 is directly coupled to the optical fiber 240, an inner surface of the sleeve 348 may have an inner diameter substantially similar to an outer diameter of the optical fiber 240.

Figures 3A and 3B illustrate exemplary arrangements wherein the optical fibers 240 are disposed against the interior sidewall 226 of the probe 210. The optical fiber 240 may be coupled to the interior sidewall 226 along a longitudinal portion thereof radially aligned with the port 222 (shown in Figures 2A-2C). Thus, the space 228 is formed within a main lumen 260 around the optical fiber 240 but for the longitudinal portion of the interior sidewall 226 to which the optical fiber 240 is coupled. Such an arrangement may enable improved aspiration of severed vitreous collagen fibers through the interior of the probe 210. The optical fiber 240 may be coupled or bonded to the interior sidewall 226 via any suitable adhesive or bonding mechanism. For example, an exterior surface of the cladding 346 or the sleeve 348 may be bonded to the interior sidewall 226 of the probe 210 with an epoxy or acrylic adhesive. However, other adhesives are also contemplated.

Figure 3C illustrates an alternative exemplary arrangement in which the optical fiber 240 is suspended within the main lumen 260. In some examples, the sleeve 348 may provide structural support and rigidity to the optical fiber 240 to enable suspension of the optical fiber 240 inside the main lumen 260 without coupling the optical fiber 240 to the interior sidewall 226. As depicted in Figure 3C, the optical fiber 240 may be centrally disposed within the main lumen 260 such that the radial distance between a point on an exterior surface of the optical fiber 240 and the interior sidewall 226 is uniform around the entire circumference of the optical fiber 240.

Figures 4A-4D illustrate exemplary front sectional views of a probe 410 having at least two optical fibers 440a, 440b housed therein. Accordingly, a first optical fiber 440a may be utilized to propagate a laser light for vitreous fibril severance and a second optical fiber 440b may be utilized to propagate an illumination light for illumination of an intraocular space. Each of the optical fibers 440a, 440b further includes a core 444a, 444b and a cladding 446a, 446b, respectively. The cores 444a, 444b and the claddings 446a, 446b may be formed of any suitable materials for propagation of laser and illumination light beams, respectively. For example, the cores 444a, 444b and the claddings 446a, 446b may comprise a transparent material such as fused silica or glass, as described above. The cores 444a, 444b and the claddings 446a, 446b may further be doped with one or more dopants depending on desired refractive properties for each of the optical fibers 440a, 440b.

The dimensions of the optical fibers 440a, 440b, including the cores 444a, 444b and claddings 446a, 446b, may be substantially similar to the dimensions of optical fiber 240, core 344, and cladding 346 described above. Although depicted as having different dimensions in Figures 4A-4D, the optical fibers 440a, 440b and the cores 444a, 444b and the claddings 446a, 446b may have similar or different dimensions to each other.

As depicted in Figures 4A and 4B, the optical fibers 440a, 440b are both disposed within a secondary lumen 462 of a sleeve 448. Similar to the sleeve 348, the sleeve 448 may provide structural support and containment of the optical fibers 440a, 440b within the main lumen 460 of the probe 410. The sleeve 448 may comprise a transparent material such as fused silica and glass. In further embodiments, the sleeve 448 is doped with a dopant to manipulate the refractive index of the sleeve 448 as desired. The sleeve 448 may have any suitable thickness to provide appropriate support and rigidity to the optical fibers 440a, 440b, such as a thickness between about 5 µm and about 50 µm, such as a thickness between about 15 µm and about 40 µm. For example, the sleeve 448 may have a thickness of about 25 µm. However, smaller or larger thicknesses are also contemplated. In some embodiments, a transparent filler material may be used within the secondary lumen 462 to prevent movement of the optical fibers 440a, 440b within. For example, an adhesive may fill all areas within the secondary lumen 462 that are not occupied by the optical fibers 440a, 440b. In other embodiments, the optical fibers 440a, 440b are disposed within the secondary lumen 462 without the utilization of a filler material.

Figures 4C and 4D illustrate alternative exemplary arrangements of the optical fibers 440a, 440b without the utilization of the sleeve 448. In Figure 4C, the optical fibers 440a, 440b are disposed within the main lumen 460 of the probe 410 without any surrounding structure other than the probe 410 itself. In some examples, the optical fibers 440a, 440b may be coupled together within the main lumen 460, such as by bonding with an adhesive. In other embodiments, the optical fibers 440a, 440b may be separate and isolated from each other within the main lumen 460. In Figure 4D, the optical fibers 440a, 440b are placed through a spacer tube 470 having one or more longitudinal bores 472 drilled therethrough to allow placement of the optical fibers 440a, 440b. The spacer tube 470 may act in a substantially similar manner to the sleeve 448 and provide structural support and containment of the optical fibers 440a, 440b. The spacer tube 470 may be formed of any suitable transparent materials, including fused silica and/or glass.

Regardless of whether the optical fibers 440a, 440b are contained within another structure in the main lumen 460, the optical fibers 440a, 440b may be arranged to either directly or indirectly contact the interior sidewall 426 or be suspended without any contact with the interior sidewall 426. Figures 4A, 4C, and 4D illustrate examples where the optical fibers 440a, 440b are either directly or indirectly coupled to the interior sidewall 426 of the probe 410. As described above, the optical fibers 440a, 440b may be directly or indirectly coupled to the interior sidewall 326 via any suitable adhesive or bonding mechanism, such as an epoxy or acrylic adhesive. Alternatively, the optical fibers 440a, 440b may be arranged within the main lumen 460 such that they do not directly or indirectly contact the interior sidewall 426, as shown in Figure 4B. Although only two optical fibers 440a, 440b are illustrated in Figures 4A-4D, more than two optical fibers may be utilized in substantially similar arrangements.

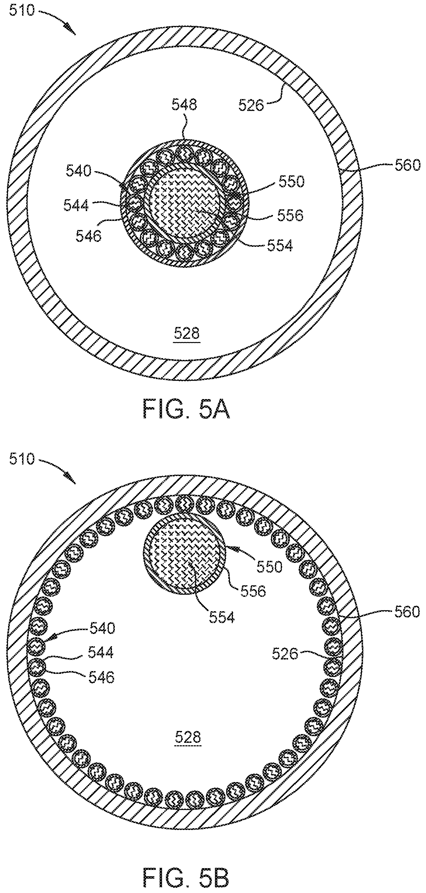

Figures 5A and 5B illustrate exemplary front sectional views of a probe 510 having a first optical fiber 550 configured to propagate a laser light and a plurality of second optical fibers 540 surrounding the first optical fiber 550 and configured to propagate illumination light. The first optical fiber 550 and the second optical fibers 540 include cores 554, 544 and claddings 556, 546, respectively. The materials and dimensions for the cores 554, 544 and the claddings 556, 546 may be substantially the same as those of the optical fibers 240 and 440 described above. However, as depicted in Figures 5A and 5B, the dimensions of the second optical fibers 540 may be smaller than that of the first optical fiber 550 to enable a sufficient volume for a space 528 within a main lumen 560 for aspiration of vitreous collagen fibers therethough.

In the exemplary arrangement of Figure 5A, a single first optical fiber 500 is disposed within the main lumen 560 and circumferentially surrounded by the plurality of second optical fibers 540 along an outer diameter thereof. In other words, a ring of second optical fibers 540 is disposed around and in contact with the cladding 556 of the first optical fiber 550 such that a center of the core 554 is equidistant or at least substantially equidistant from centers of the cores 544. In further embodiments, an optional sleeve 548 is disposed around the plurality of second optical fibers 540 and configured to tightly hold the second optical fibers 540 against the first optical fiber 550. The sleeve 548 may have any suitable thickness and dimensions to ensure that the second fibers 540 are tightly packed together and there is no room for the second fibers 540 to be loose or move. Although depicted as being centrally suspended within the main lumen 560, the first optical fiber 550 and surrounding second optical fibers 540 may be disposed in any suitable location within the main lumen 560. For example, the first optical fiber 550 and surrounding second optical fibers 540 may be coupled to an interior sidewall 526 of the probe 510 along a longitudinal portion radially aligned with the port 222.

Figure 5B illustrates another exemplary arrangement of the single first optical fiber 550 and the plurality of second optical fibers 540 within the main lumen 560. Unlike Figure 5A, the second optical fibers 540 are arranged along the interior sidewall 526 and not along an outer diameter of the laser fiber 500. Thus, although the second optical fibers 540 are still surrounding the first optical fiber 550, the center of the core 554 is not equidistant or substantially equidistant from the centers of the cores 544. Furthermore, vitreous collagen fibers may be aspirated in the space 528 located radially inward of the second optical fibers 540. In some embodiments, the first optical fiber 550 is suspended within the main lumen 560 such that it does not contact any of the second optical fibers 540 lining the interior sidewall 526. In the embodiment of Figure 5B, however, the first optical fiber 550 is disposed against one or more second optical fibers 540 and thus, the first optical fiber 550 is indirectly coupled to the interior sidewall 526 by the one or more second optical fibers 540. The arrangements of the first optical fiber 550 and second optical fibers 540 depicted in Figures 5A and 5B may provide improved illumination of the intraocular space during use thereof due to the 360-degree arrangement of the second optical fibers 540 around the first optical fiber 550.

In addition to utilizing different arrangements of optical fibers within the probe of a surgical instrument, the propagation of illumination light into an operating region (e.g., the intraocular space) may be modified by the utilization of different materials for the probe and/or by use of a mask. For example, the probe may include one or more sections formed of a translucent or transparent material and one or more sections formed of an opaque or semi-opaque material. In some embodiments, the distal portion of the probe is formed of a translucent or transparent material while the proximal portion of the probe is formed of a metal, such as stainless steel or aluminum. In some embodiments, only the distal tip of the probe (including the area around the port) is formed of a translucent or transparent material, and the remainder of the distal portion and the entirety of the proximal portion are formed of metal. In further embodiments, both the distal portion and the proximal portion of the probe are entirely formed of a translucent or transparent material.

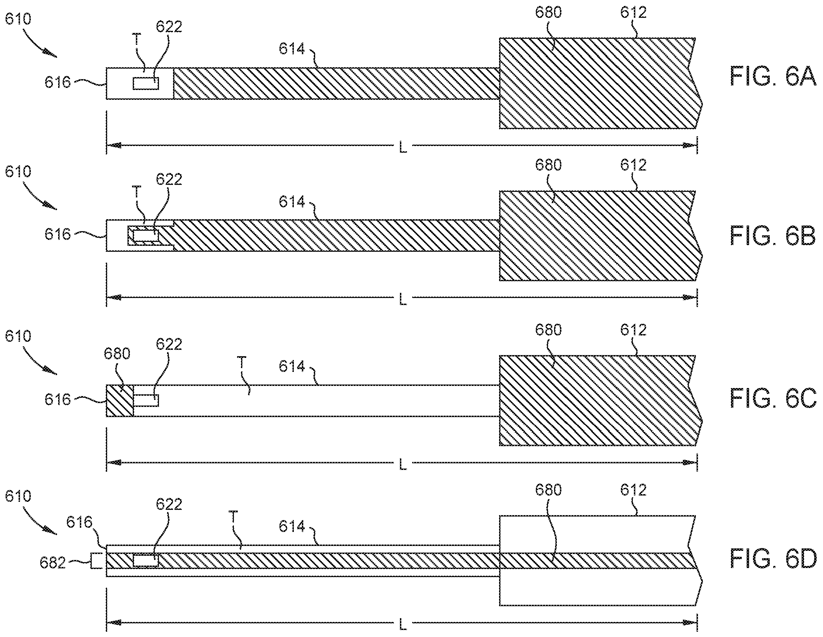

Figures 6A-6D illustrate plan views of the exterior of a probe 610 having different exemplary arrangements of a mask 680 to modify the propagation of illumination light through a distal portion 614 and a proximal portion 612 otherwise formed of a translucent or transparent material. The mask 680 refers to any suitable device, material, or mechanism for reducing or preventing transmission of illumination light therethrough. For example, the mask 680 may refer to areas of the probe 610 having a higher refractive index relative to other areas of the probe 610. In some examples, the mask 680 refers to opaque or semi-opaque portions of the probe 610 through which reduced or no illumination light may pass. In other examples, the mask 680 refers to an opaque or semi-opaque film or layer applied to an exterior or interior surface of the probe 610. In still other examples, the mask 680 refers to metal portions of the probe 610. In any form, the mask 680 may be disposed in any suitable arrangement along a length L of the probe 610 to provide optimal illumination of the intraocular space and reduce glare for a user of a surgical instrument caused by the illumination light.

In one exemplary embodiment depicted in Figure 6A, the mask 680 substantially surrounds the proximal portion 612 and the distal portion 614 of the probe 610, but for a translucent or transparent portion T adjacent to a port 622. The area T begins proximal to the port 622 and terminates at a distal tip 616, circumferentially wrapping around the probe 610. Accordingly, the transmission of illumination light from the one or more optical fibers within the probe 610 to the surrounding environment is substantially reduced or prevented along the majority of the length L covered by the mask 680 and proximal relative to the translucent or transparent portion T. Rather, the majority of illumination light emitted from the probe 610 in Figure 6A may be propagated radially outward 360° from the translucent or transparent portion T. Accordingly, illumination during use of the probe 610 may be limited to an area within the operating region (e.g., the intraocular space) adjacent to the port 622 through which vitreous material may be severed and aspirated, providing improved visibility with reduced glare for a user thereof.

Figure 6B depicts another exemplary embodiment substantially similar to that of Figure 6A where the mask 680 further extends around the port 622. For example, the mask 680 may form a perimeter around (e.g., surrounding) the port 622, and the port 622 may be described as being "fenced in" by the mask 680. The additional masking around the port 622 may further reduce glare caused by illumination light being transmitted through the sidewalls of probe 610 and improve visibility of the intraocular space.

In the exemplary embodiment depicted in Figure 6C, the distal portion 614 of the probe 610 is substantially transparent but for an area covered by the mask 680 at the distal tip 616. The covered area begins at a distal end of the port 622 and envelopes the distal tip 616 of the probe 610. During vitrectomy, the distal tip 616 of the probe 610 is often placed in close proximity to the retina of a patient's eye, and excess illumination through the distal tip 616 may cause retinal damage. Thus, utilization of the masking arrangement in Figure 6C may reduce or eliminate retinal damage caused by illumination light being transmitted near the patient's retina, and illumination light may only be transmitted through the translucent or transparent portion T and radially outward from the probe 610 between the distal end of the port 622 and the proximal portion 612.

Figure 6D depicts another exemplary embodiment of a masking arrangement for the probe 610. As depicted in Figure 6D, the mask 680 only covers a longitudinal quadrant 682 of the distal portion 614 and continues along the length L of the probe 610 across the proximal portion 612. The longitudinal quadrant 682 covered by the mask 680 encompasses the port 622 and thus, no illumination light is emitted from the probe 610 along a user's line of sight along the probe 610 towards the port 622. Accordingly, illumination light may be transmitted radially outward from the probe 610 in a direction away from the user's line of sight when performing vitreoretinal surgery, thus reducing or eliminating any interfering glare. Although depicted as only covering the longitudinal quadrant 682, it is also contemplated that the mask 680 may cover greater or lesser areas of the probe 610, such as a longitudinal hemisphere of the probe 610.

In summary, embodiments of the present disclosure include devices and structures for performing vitreoretinal surgery. In particular, the surgical instruments described above combine the functions of laser vitrectomy and intraocular illumination, thus enabling more efficient performance of vitreous removal. Utilization of a laser vitrectomy probe allows the collagen fibers of vitreous material to be easily removed, which reduces retinal traction produced by removing the vitreous material. Furthermore, propagation of illumination light through a vitrectomy probe having portions thereof formed of translucent materials enables diffuse intraocular illumination without the need for a secondary illumination device, which may provide inefficient intraocular illumination or limit the operating room within the intraocular space. Still further, the embodiments described herein provide arrangements reducing the occurrence of glare for a user of a vitrectomy probe, which is a common problem for conventional ophthalmic illuminators. Accordingly, the described embodiments enable the performance of more efficient, less invasive, and safer vitreoretinal procedures.

Although vitreous surgery is discussed as an example of a surgical procedure that may benefit from the described embodiments, the advantages of the surgical devices and systems described herein may benefit other surgical procedures as well.

Examples of embodiments disclosed herein include a surgical instrument, comprising: a base unit; a probe disposed through an opening in a distal end of the base unit, the probe comprising: a port formed proximate to a distal tip of the probe; a lumen formed through the probe; and one or more optical fibers disposed in the lumen, the optical fibers projecting a laser light for irradiating an area proximate to the port to cut collagen fibers of vitreous material aspirated through the port, the one or more optical fibers further projecting an illumination light for illumination of an intraocular space of a patient. The one or more optical fibers may comprise a single optical fiber configured to project the laser light and the illumination light. The laser light may be focused on a core of the optical fiber and the illumination light may be focused on a cladding of the optical fiber. The illumination light may also be focused on the core of the optical fiber. The one or more optical fibers may comprise a first optical fiber configured to project laser light and a second optical fiber configured to project illumination light. The illumination light may be focused on a core or cladding (or both) of the second optical fiber. The laser light may be a continuous or pulsed laser light. The pulsed laser light may be a picosecond or femtosecond laser light. The laser light and the illumination light may be coaxially projected from the one or more optical fibers. The probe may further comprise one or more sections formed of a translucent or transparent material to facilitate transmission of illumination light therethrough. The illumination light may be emitted radially outward from the optical fiber and transmitted through the translucent or transparent material of the probe. The probe may further comprise an opaque material to reduce transmission of illumination light therethrough and reduce glare caused by the illumination light. A portion of the probe adjacent to the port may be formed of the translucent or transparent material and a remainder of the probe may comprise the opaque material. The opaque material may further form a perimeter around the port. A longitudinal quadrant along a length of the probe may comprise the opaque material and the remainder of the probe may be formed of the translucent or transparent material. The laser light and the illumination light may be simultaneously projected through the one or more optical fibers. The laser light and the illumination light may be sequentially pulsed through the one or more optical fibers. The one or more optical fibers may be suspended within the lumen such that an aspiration space circumferentially surrounds the one or more optical fibers. The one or more optical fibers may be coupled to a sidewall of the lumen and aligned with the port.

While the foregoing is directed to embodiments of the present disclosure, other and further embodiments of the disclosure may be devised without departing from the basic scope thereof, and the scope thereof is determined by the claims that follow.

## Claims

1. A surgical instrument (100), comprising:
a base unit (120);
a probe (110) disposed through an opening in a distal end (121) of the base unit, the probe comprising:
a port (222) formed in a sidewall of the probe and proximate to a distal tip (216) of the probe;
a lumen (260) formed through the probe;
one or more optical fibers (240, 440) disposed in the lumen (260), the one or more optical fibers comprising a first optical fiber (440a) configured to project a laser light (241) produced by a laser light source (264) for irradiating an area proximate to the port (222) to cut collagen fibers of vitreous material aspirated through the port (222), the one or more optical fibers further comprising a second optical fiber (440b) configured to project an illumination light (243) produced by an illumination light source (266) for illumination of an intraocular space of a patient; **characterised in that** the first optical fiber and the second optical fiber are each coupled to the sidewall of the lumen and are aligned with the port.

2. The surgical instrument of claim 1, wherein the illumination light (243) is focused on a cladding (446b) of the second optical fiber (440b).

3. The surgical instrument of claim 1, wherein the illumination light is focused on a core (444b) of the second optical fiber (440b).

4. The surgical instrument of claim 2, wherein the illumination light is also focused on a core (444b) of the second optical fiber (440b).

5. The surgical instrument of claim 1, wherein the laser light (241) is a pulsed laser light.

6. The surgical instrument of claim 5, wherein the pulsed laser light is a picosecond laser light.

7. The surgical instrument of claim 5, wherein the pulsed laser light is a femtosecond laser light.

8. The surgical instrument of claim 1, wherein the laser light is a continuous laser light.

9. The surgical instrument of claim 1, wherein the illumination light is a pulsed illumination light.

10. The surgical instrument of claim 1, wherein the laser light (241) and the illumination light (243) are coaxially projected from the one or more optical fibers (240, 440).

11. The surgical instrument of claim 1, wherein the probe (110) further comprises one or more sections formed of a translucent or transparent material to facilitate transmission of illumination light (243) therethrough.

## Patentansprüche

1. Chirurgisches Instrument (100), umfassend:
eine Basiseinheit (120);
eine Sonde (110), die durch eine Öffnung in einem distalen Ende (121) der Basiseinheit hindurch angeordnet ist, wobei die Sonde Folgendes umfasst:
einen Anschluss (222), der in einer Seitenwand der Sonde und nahe einer distalen Spitze (216) der Sonde gebildet ist;
ein Lumen (260), das durch die Sonde hindurch gebildet ist;
eine oder mehrere optische Fasern (240, 440), die in dem Lumen (260) angeordnet sind, wobei die eine oder mehreren optischen Fasern eine erste optische Faser (440a) umfassen, die dazu ausgelegt ist, ein Laserlicht (241) zu projizieren, das durch eine Laserlichtquelle (264) produziert wird, um einen Bereich nahe dem Anschluss (222) zu bestrahlen, um Kollagenfasern aus Glaskörpermaterial zu schneiden, die durch den Anschluss (222) aspiriert werden, wobei die eine oder mehreren optischen Fasern ferner eine zweite optische Faser (440b) umfassen, die dazu ausgelegt ist, ein Beleuchtungslicht (243) zu projizieren, das durch eine Beleuchtungslichtquelle (266) produziert wird, um einen intraokularen Raums eines Patienten zu beleuchten; **dadurch gekennzeichnet, dass** die erste optische Faser und die zweite optische Faser jeweils an die Seitenwand des Lumens gekoppelt sind und mit dem Anschluss ausgerichtet sind.

2. Chirurgisches Instrument nach Anspruch 1, wobei das Beleuchtungslicht (243) auf einen Mantel (446b) der zweiten optischen Faser (440b) fokussiert ist.

3. Chirurgisches Instrument nach Anspruch 1, wobei das Beleuchtungslicht auf einen Kern (444b) der zweiten optischen Faser (440b) fokussiert ist.

4. Chirurgisches Instrument nach Anspruch 2, wobei das Beleuchtungslicht auch auf einen Kern (444b) der zweiten optischen Faser (440b) fokussiert ist.

5. Chirurgisches Instrument nach Anspruch 1, wobei das Laserlicht (241) ein gepulstes Laserlicht ist.

6. Chirurgisches Instrument nach Anspruch 5, wobei das gepulste Laserlicht ein Pikosekundenlaserlicht ist.

7. Chirurgisches Instrument nach Anspruch 5, wobei das gepulste Laserlicht ein Femtosekunden-Laserlicht ist.

8. Chirurgisches Instrument nach Anspruch 1, wobei das Laserlicht ein kontinuierliches Laserlicht ist.

9. Chirurgisches Instrument nach Anspruch 1, wobei das Beleuchtungslicht ein gepulstes Beleuchtungslicht ist.

10. Chirurgisches Instrument nach Anspruch 1, wobei das Laserlicht (241) und das Beleuchtungslicht (243) koaxial von der einen oder den mehreren optische Fasern (240, 440) projiziert werden.

11. Chirurgisches Instrument nach Anspruch 1, wobei die Sonde (110) ferner einen oder mehrere Abschnitte umfasst, die aus einem durchscheinenden oder transparenten Material gebildet sind, um die Übertragung von Beleuchtungslicht (243) durch sie hindurch zu erleichtern.

## Revendications

1. Instrument chirurgical (100), comprenant :
une unité de base (120) ;
une sonde (110) disposée à travers une ouverture d'une extrémité distale (121) de l'unité de base, la sonde comprenant :
un orifice (222) formé dans une paroi latérale de la sonde et à proximité d'une pointe distale (216) de la sonde ;
une lumière (260) formée à travers la sonde ;
une ou plusieurs fibres optiques (240, 440) disposées dans la lumière (260), la ou les fibres optiques comprenant une première fibre optique (440a) configurée pour projeter une lumière laser (241) produite par une source de lumière laser (264) pour irradier une zone proche de l'orifice (222) pour couper des fibres de collagène de matériau vitreux aspirées à travers l'orifice (222), la ou les fibres optiques comprenant en outre une seconde fibre optique (440b) configurée pour projeter une lumière d'éclairage (243) produite par une source de lumière d'éclairage (266) pour éclairer un espace intraoculaire d'un patient ; **caractérisée en ce que** la première fibre optique et la seconde fibre optique sont chacune couplées à la paroi latérale de la lumière et sont alignées avec l'orifice.

2. Instrument chirurgical selon la revendication 1, la lumière d'éclairage (243) étant focalisée sur une gaine (446b) de la seconde fibre optique (440b).

3. Instrument chirurgical selon la revendication 1, la lumière d'éclairage étant focalisée sur un noyau (444b) de la seconde fibre optique (440b).

4. Instrument chirurgical selon la revendication 2, la lumière d'éclairage étant également focalisée sur un noyau (444b) de la seconde fibre optique (440b).

5. Instrument chirurgical selon la revendication 1, la lumière laser (241) étant une lumière laser pulsée.

6. Instrument chirurgical selon la revendication 5, la lumière laser pulsée étant une lumière laser picoseconde.

7. Instrument chirurgical selon la revendication 5, la lumière laser pulsée étant une lumière laser femtoseconde.

8. Instrument chirurgical selon la revendication 1, la lumière laser pulsée étant une lumière laser continue.

9. Instrument chirurgical selon la revendication 1, la lumière d'éclairage étant une lumière d'éclairage pulsée.

10. Instrument chirurgical selon la revendication 1, la lumière laser (241) et la lumière d'éclairage (243) étant projetées coaxialement à partir de la ou des fibres optiques (240, 440).

11. Instrument chirurgical selon la revendication 1, la sonde (110) comprenant en outre une ou plusieurs sections formées d'un matériau translucide ou transparent pour faciliter la transmission de la lumière d'éclairage (243) à travers celle-ci.
